(19) Europäisches Patentamt
European Patent Office
Office européen des brevets

(11) Numéro de publication : **0 326 455 B1**

# (12) FASCICULE DE BREVET EUROPEEN

(45) Date de publication du fascicule du brevet : **13.05.92 Bulletin 92/20**

(51) Int. Cl.$^5$ : **C07C 50/24,** C07C 46/00

(21) Numéro de dépôt : **89400118.9**

(22) Date de dépôt : **16.01.89**

## (54) Procédé de préparation de chloranil.

(30) Priorité : **27.01.88 FR 8800911**

(43) Date de publication de la demande : **02.08.89 Bulletin 89/31**

(45) Mention de la délivrance du brevet : **13.05.92 Bulletin 92/20**

(84) Etats contractants désignés : **AT BE CH DE ES FR GB GR IT LI LU NL SE**

(56) Documents cités :
**DD-A- 9 531**
**DE-A- 2 645 114**
**US-A- 2 422 229**
**US-A- 3 396 178**
**BEILSTEINS HANDBUCH DER ORGANISCHEN CHEMIE, Tome 7, 3 supplément, 4 partie, page 3378, Springer Verlag, Berlin, 1969**

(56) Documents cités :
**CHEMICAL ABSTRACTS, Tome 31, no. 19, 10 octobre 1937, Columbus, Ohio, US; V. V. CHELINTSEV et al, "Oxonium compounds.Complexes of quinones with hydrochloric, phosphoric and acetic acids and their chlorination"**
**CHEMIKER-ZEITUNG, Tome 56, no. 58, 20 juillet 1932, Köthen, D; R. SCHULOFF: "Ueber zwei neue Verfahren zur Herstellung vonChloranil im Laboratorium und Betriebe"**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière Route de Ternay**
**F-69360 Communay (FR)**
Inventeur : **Jouve, Isabelle**
**30, rue Léon Fabre**
**F-69100 Villeurbanne (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

EP 0 326 455 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de chloranil à partir d'hydroquinones ou de quinones éventuellement en partie chlorées pouvant être mises en oeuvre dans un appareillage le plus simple possible.

Le chloranil est un intermédiaire de synthèse fort connu et utilisé dans la synthèse de matières colorantes. Les procédés de synthèse décrits dans la littérature d'un tel composé sont nombreux, les rendements annoncés sont pour certains procédés excellents, de l'ordre de 95 % et même supérieurs.

Il a par exemple été décrit, il y a déjà très longtemps par R. Schuloff, R. Pollak dans Chemiker-Zeitung 56, p. 569 (1932) la chloration de la quinone en présence de solutions concentrées d'acide chlorhydrique. La chloration est effectuée à haute température pendant des durées de réaction très longues. A cause de la température élevée et des quantités importantes de chlore qui sont utilisées, il y a d'une part sublimation d'une partie du chloranil qu'il faut condenser pour le récupérer et d'autre part une forte pollution due au chlore excédentaire n'ayant pas réagi.

Il est aussi connu de nombreux procédés de chloration utilisant l'acide chlorhydrique concentré en présence de catalyseurs variés. Il est par exemple décrit dans le brevet allemand 2 645 114 un procédé de chloration oxydante de l'hydroquinone en présence de dichlorure de magnésium, dans la publication effectuée par Fischer et Henderson parue dans Synthesis 1985, (6.7), 641-3., l'oxydation est effectuée en présence de nitrate de cérium, dans la publication effectuée par Rettig et Latscha (Z. Naturforsch, 35 b, 399-400 (1980)) la chloration est effectuée en présence de pentachlorure d'antimoine.

L'industrie a toujours cherché à éviter l'utilisation de catalyseurs de chloration dans tout procédé pouvant fonctionner en l'absence de ceux-ci.

Ainsi il a été proposé dans le brevet EP 220 135 un procédé de chloration oxydante d'hydroquinone ou de quinone en l'absence de tout catalyseur, le procédé étant caractérisé par l'utilisation d'une pression élevée de chlore comprise entre 3 et 40 bar. La concentration de l'hydroquinone dans l'acide chlorhydrique ne dépasse jamais 70 g/litre ce qui est faible pour un procédé industriel, en outre la pression utilisée exige un appareillage spécial.

Il a également été décrit, par V.V. CELINCEV [Comptes-rendus de l'Académie de l'URSS 14 p. 289 et suivantes (1937)], un procédé de préparation de chloranil qui consiste à effectuer la chloration de la quinone, au moyen du mélange $Cl_2$/HCl sec, dans l'acide acétique concentré. Toutefois, un tel procédé nécessite des conditions anhydres qui imposent toujours des contraintes industrielles puisqu'il est impératif d'effectuer une déshydratation préalable de l'agent de chloration.

La préparation du chloranil à partir de sources phénoliques a été abandonnée par toute l'industrie chimique car ils forment au cours de la chloration oxydante des sous produits du type dioxine dont la toxicité est telle que leur présence doit être à tout prix évitée (décret allemand sur les substances dangereuses de septembre 1986 § 9 (6)).

La présente invention a permis de résoudre les problèmes laissés par l'art antérieur, elle permet de s'affranchir de la présence de catalyseurs halogénométalliques, elle permet de travailler à la pression atmosphérique, elle permet en dernier de travailler avec de fortes concentrations en matières premières de l'ordre de 100 g par litre réactionnel.

L'invention est caractérisée par le fait qu'on réalise une chloration oxydante d'une quinone ou d'une hydroquinone éventuellement chlorée dans un solvant miscible à l'eau ou à l'acide chlorhydrique en l'absence de catalyseur de chloration et à la pression atmosphérique.

La quinone éventuellement chlorée répond à la formule (I) suivante

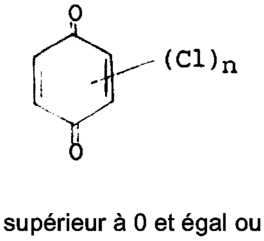

(I)

dans laquelle n est égal ou supérieur à 0 et égal ou inférieur à 3.

L'hydroquinone éventuellement chlorée répond à la formule (II) suivante

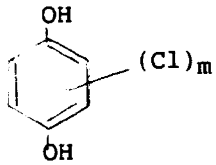

(II)

dans laquelle m est égal ou supérieur à 0 et égal ou inférieur à 4.

On préfère utiliser comme matière première l'hydroquinone non substituée car elle représente la matière première la moins onéreuse.

L'agent de chloration oxydante est choisi parmi le chlore ou le mélange acide chlorhydrique-péroxyde d'hydrogène. Le chlore peut être utilisé soit en présence d'eau soit en présence d'acide chlorhydrique mélangé à un solvant organique miscible soit même directement dans le solvant organique. Même si l'on débute la réaction en présence d'eau, elle se déroulera néanmoins en présence de l'acide chlorhydrique forme au cours de la réaction.

On utilise de préférence un milieu saturé en acide chlorhydrique ce qui se fera naturellement lors de l'introduction de chlore gazeux.

Dans le cas de l'utilisation du mélange acide chlorhydrique-péroxyde d'hydrogène la saturation pourra être maintenue par addition d'acide chlorhydrique gazeux ou de chlore.

Le solvant utilisé est un solvant miscible avec l'eau ou avec l'acide chlorhydrique gazeux ou en solution aqueuse. Il est choisi parmi les alcools contenant 1 à 4 atomes de carbone, les acides carboxyliques contenant 1 à 4 atomes de carbone ou leurs anhydrides, l'éther isopropylique, les amides, les nitriles, le diméthylsulfoxide.

Parmi les alcools on peut citer : le méthanol, l'éthanol, l'isopropanol, le butanol.

Parmi les acides carboxyliques on peut citer l'acide formique, l'acide acétique, l'acide propionique, l'acide butyrique ainsi que leurs anhydrides.

Parmi les nitriles on peut cite l'acétonitrile, le propionitrile.

Parmi les amides on peut citer : le diméthylformamide, la N méthylpyrrolidone.

On préfère utiliser l'acide acétique ou l'éthanol.

La quantité de chlore utilisée varie avantageusement entre la stoechiométrie et un excès de 20 % par rapport à celle-ci. Un excès d'environ 10 % est tout à fait conseillé.

Lorsque l'on utilise un mélange acide chlorhydrique-péroxyde d'hydrogène, on préfère utiliser une solution d'acide chlorhydrique environ 10N (30 à 37 % en poids). On met oeuvre un large excès d'acide chlorhydrique de l'ordre de 10 à 50 moles d'acide chlorhydrique par mole d'hydroquinone.

Le péroxyde d'hydrogène utilisé est de préférence une solution aqueuse la plus concentrée possible de l'ordre de 30 % en poids par exemple.

On utilise au moins 5 moles de péroxyde d'hydrogène par mole d'hydroquinone et de préférence un excès de 20 % est conseillé.

Le solvant est utilisé de préférence selon un rapport volumétrique solvant/solution chlorhydrique supérieur à 0,5 et encore plus préférentiellement supérieur ou égal à 1.

La quinone ou l'hydroquinone est introduite dans le milieu réactionnel acide chlorhydrique ou eau et/ou au solvant à une concentration de préférence inférieure à 130-140 g/litre. Une concentration de l'ordre de 110 g est tout à fait conseillée.

La température réactionnelle est avantageusement comprise entre 50°C et le reflux. Une température d'environ 90°C est préférée. La pression utilisée est la pression atmosphérique.

Les exemples suivants permettront de mieux comprendre l'invention. Ils ne sont donnés qu'à titre illustratif et ne doivent pas être considérés comme limitatifs de l'invention.

Les abréviations suivantes seront utilisées :

- HQ : hydroquinone
- $Cl_3BQ$ : trichlorobenzoquinone
- $Cl_4HQ$ : tétrachlorohydroquinone
- RR : rendement sur le produit introduit.

Essai comparatif selon le brevet DE 2 645 114

Dans un réacteur de 500 ml muni d'une agitation centrale à pales, d'un réfrigérant, d'une ampoule de coulée, d'un thermomètre, on charge 200 ml d'acide chlorhydrique à 35 % (2,28 M), 17.63 g de $MgCl_2$, $6H_2O$ (0,086 M), 2,82 g d'hydroquinone (0,0256 M).

Après avoir porté le mélange réactionnel à 0°, 15 ml d'eau oxygénée à 30 % (0,146 M) sont coulés en 15

minutes. Le mélange est ensuite porté à 100° en 1 h 30 ce qui provoque la formation de mousse montant dans le réfrigérant. Après deux heures de chauffage à 100°, le mélange est refroidi, puis le précipité est recueilli par filtration sur un filtre-fritte. Le précipité est lavé par 3 x 20 ml d'eau et séché à l'étuve. Le précipité recueilli pèse 5,89 g soit pondéralement 93,6 % du chloranil attendu. Le dosage chromatographie phase liquide de ce produit montre qu'il contient 49,2 % de chloranil et 44,8 % de tétrachlorobenzoquinone ce qui conduit respectivement à des RR de 46,2 % et de 41,7 %. Le produit brut contient également 6 % de chlorure de magnésium.

Exemples 1 à 3

Dans le même réacteur que pour l'exemple comparatif, on met en oeuvre :

– 250 ml d'acide chlorhydrique à 36 % (2,71 mole),
– 3,75 g d'hydroquinone (34 mmoles).

Dans cette solution chauffée à 90°C on introduit en 120′ 23,18 g de péroxyde d'hydrogène à 30 % (0,204 moles).

On laisse agiter quelques minutes. Le mélange réactionnel est refroidi jusqu'à température ambiante, il est filtré. Après séchage on recueille 7,75 g de poudre jaune ce qui représente un rendement pondéral de 92,8 % par rapport au chloranil attendu. L'analyse chromatographie liquide conduit à la composition suivante :

- chloranil : 89,4 %
- $Cl_3BQ$ : 5,3 %
- $Cl_4HQ$ : 5 %.

On opère dans les mêmes conditions que précédemment en utilisant 250 ml d'une solution HCl éthanol à 18 % en volume d'éthanol et de 2 solutions HCl-acide acétique à 32 et 57 % en volume d'acide acétique. Les résultats sont reportés dans le tableau 1.

Exemples 4 et 5 :

. Mode opératoire avec $HCl/H_2O_2$

Dans un réacteur de 2 litres muni d'une agitation centrale à pales, d'un réfrigérant, d'une ampoule de coulée, d'un thermomètre, on charge 400 ml d'acid chlorhydrique à 37 % (4,76 M), 400 ml d'acide acétique, 27,2 g d'hydroquinone (0,247 M). Le mélange est chauffé à 70°, on commence à introduire 150 ml d'eau oxygénée à 30 % ce qui représente un excès molaire de 20 % (1,46 Mole) tout en continuant à chauffer la masse réactionnelle jusqu'à 95 - 100°. L'addition d'eau oxygénée est réalisée en deux heures. Après refroidissement, le chloranil est filtré, séché pour être ensuite analysé en chromatographie liquide haute performance (voir résultat sur tableau 2).

. Mode opératoire avec $Cl_2/HCl$

Dans un réacteur de 2 litres muni d'une agitation centrale à pales, d'une réfrigérant, d'un thermomètre, d'un tube plongeur pour l'arrivée du chlore, on charge 400 ml d'acide chlorhydrique à 37 % (4,76 M), 400 ml d'acide acétique, 27,2 g d'hydroquinone (0,247 M). Le mélange est chauffé à 70°, on commence à introduire le chlore. Les 33,5 litres de chlore (1,49 M) sont introduits en 5 h 30, après refroidissement, le chloranil est filtré, séché pour être analysé en chromatographie liquide haute performance (voir résultats sur tableau 2).

Exemples 6 à 8

Influence de la concentration en hydroquinone.

Afin d'accroître la productivité de la chloration, nous avons examiné l'influence de la concentration en hydroquinone. Le débit de chlore dans ces essais a également varié pour maintenir une durée de chloration de 5 h 30 avec un excès de 20 % en $Cl_2$ pour un milieu AcOH-HCl (50/50) (voir tableau 3).

Avec une concentration initiale de 110 g/litre, la concentration en chloranil finale est de 245,9 g/litre.

Exemples 9 et 10

Introduction de dichlorobenzoquinone ($Cl_2BQ$).

Exemple 9

Dans un réacteur on charge 7,2 g de dichlorobenzoquinone ($Cl_2BQ$) (40 mmoles) dans 38 ml d'acide acétique et 38 ml d'HCl à 37 %.

On chauffe à 90°C. On introduit à cette température du chlore à un débit de 3 l/h pendant 35 minutes (80 mmoles). On refroidit. On filtre un solide jaune de 9,8 g (99 %) dont la composition est indiquée dans le tableau 4.

Exemple 10

On procède au même exemple que précédemment, on introduit :

– 3 g de dichlorobenzoquinone (17 mmoles),
– 15 ml d'acide acétique,
– 15 ml d'HCl à 37 %.

On introduit alors à 90°C en 45 minutes, 4,2 ml de péroxyde d'hydrogène à 70 % (41 mmoles). On chauffe 10 minutes et on récupère comme précédemment 4,05 g d'un solide jaune (97 %) dont la composition est indiquée dans le tableau 4.

Exemple 11

Recyclage du milieu réactionnel.

Nous avons examiné le recyclage des solvants de la masse réactionnelle.

Essai de base

Cet essai décrit ci-avant (exemple 8) a conduit aux résultats du tableau 5.

Après filtration, le filtrat (350 ml) a été récupéré et nous avons fait un appoint d'un mélange de 50:50 d'acide acétique et d'acide chlorhydrique de façon à compléter à 500 ml.

Recyclage

Nous avons refait un essai dans le mêmes conditions et avec les mêmes charges que l'essai de base en introduisant la solution réactionnelle de l'essai de base complétée à 500 ml. Ceci a conduit aux résultats du tableau 5.

Exemple 12

On opère comme dans l'exemple 5 en chargeant

29,2 g d'hydroquinone (0,265 ml),
280 g d'acide acétique soit 270 ml,
70 g d'eau soit 70 ml,
106 g de $Cl_2$ (1,49 mole) soit 113 % de la stoechiométrie.

Le chlore est additionné entre 93 et 97°C à un débit de 20 g/h jusqu'à précipitation du milieu, puis à 10 g/h jusqu'à la stoechiométrie c'est-à-dire 5 équivalents et à raison de 5 g/h pour la quantité correspondant à l'excès de chlore.

Le mélange réactionnel est agité 1 heure à 95 - 97°C puis est refroidi à 20°C. Le chloranil est filtré, essoré, lavé par 4 x 200 ml d'eau de manière à éliminer l'acidité et les $Cl^-$. Le chloranil est séché sous vide à 60°.

Les eaux-mères sont recyclées à l'opération suivante sans complément et ainsi de suite.

Les résultats figurent dans le tableau 6.

TABLEAU 1

| Essai | Concentration HQ initiale | Excès $H_2O_2$ | Composition | | Durée | T | Rdt produit isolé | Composition | | |
|---|---|---|---|---|---|---|---|---|---|---|
| | | | % HCl 36 % | % Ethanol ou acide acétique | | | | Chloranil | $Cl_3BQ$ | $Cl_4HQ$ |
| Comparatif | 1,5 g/l | 20 % | 100 | | 120' | 90° | 92,8 % | 89,4 % | 5,3 % | 5 % |
| 1 | 2,3 g/l | 20 % | 82 | 18 (EtOH) | 120' | 90° | 92,6 % | 97 % | 3 % | 0,5 % |
| 2 | 1,5 g/l | 20 % | 63 | 32 (ACOH) | 120' | 90° | 98 % | 95 % | 2 % | 1 % |
| 3 | 1,5 g/l | 20 % | 43 | 57 (ACOH) | 120' | 90° | 97 % | 97 % | 3 % | 0,8 % |

TABLEAU 2

| Essai | Agent chloranil | Poids obtenu | Rdt pondéral | Composition | | | RR chloranil |
|---|---|---|---|---|---|---|---|
| | | | | Chloranil | $Cl_3BQ$ | $Cl_4HQ$ | |
| 4 | $H_2O_2/HCl$ | 58,54 g | 96,3 % | 97 % | 3,0 % | 1,1 % | 93,4 % |
| 5 | $Cl_2$ | 61,25 g | 98,6 % | 99 % | 0,1 % | 0,6 % | 97,6 % |

TABLEAU 3

| Essais | $Cl_2$ Débit pour 1 l de solution | Concentration hydroquinone | Rdt pondéral | Composition | | | RR chloranil |
|---|---|---|---|---|---|---|---|
| | | | | Chloranil | $Cl_3BQ$ | $Cl_4HQ$ | |
| 6 | 7,4 l/h | 34 g/l | 97,7 % | 99 % | < 0,1 % | 0,5 % | 96,7 % |
| 7 | 15 l/h | 68 g/l | 97,6 % | 99,4 % | 0,1 % | 0,4 % | 97 % |
| 8 | 24 l/h | 110 g/l | 97,6 % | 99,5 % | 0,2 % | 0,7 % | 97,1 % |

TABLEAU 4

| Essais | Agent de chloration | Concentration $Cl_2BQ$ introduit | Rdt pondéral | Composition | | | RR chloranil |
|---|---|---|---|---|---|---|---|
| | | | | chloranil | $Cl_3BQ$ | $Cl_4HQ$ | |
| 9 | $Cl_2$ | 100 g/l | 99 % | 98,4 % | 0,2 % | 1,4 % | 97,4 % |
| 10 | $HCl/H_2O_2$ | 100 g/l | 97 % | 96,2 % | 3,2 % | 0,6 % | 93,3 % |

TABLEAU 5

| Essais | Concentration HQ | Excès $Cl_2$ | Rdt pondéral | Composition | | | |
|---|---|---|---|---|---|---|---|
| | | | | Chloranil | $Cl_3BQ$ | $Cl_4HQ$ | RR Chloranil |
| De base | 110 g/l | 20 % | 97,6 % | 99,5 % | 0,2 % | 0,7 % | 97,1 % |
| Recyclage | 110 g/l | 20 % | 97,3 % | 99 % | 0,4 % | 0,6 % | 95,7 % |

TABLEAU 6

| | | 1ère opération | 2ème opération | 3ème opération |
|---|---|---|---|---|
| HQ engagé en g | | 29,2 | 29,2 | 29,2 |
| Milieu réactionnel | $CH_3CO_2H$ | 280 | | |
| | $H_2O$ | 70 | | |
| | HCl | 0 | | |
| | Masse | 350 | 347 | 314 |
| | Volume | 340 | 315 | 280 |
| $Cl_2$ engagé | Masse | 106 | 112 | 114 |
| | Stoechiométrie | 113 % | 119 % | 121 % |
| | Durée totale addition | 6 H | 6 H 30 | 7 H 10 |
| Chloranil récupéré | | 58 g | 65,4 g | 66 g |
| Rendement pondéral | | 89 % | 100,4 % | 101,2 % |
| Titre chloranil | | › 99 % | › 99 % | › 99 % |

## Revendications

1. Procédé de préparation du chloranil par chloration ou chloration oxydante de quinones, d'hydroquinones

ou de leurs dérivés chlorés en l'absence de catalyseur de chloration et à la pression atmosphérique caractérisé en ce que la chloration est effectuée à l'aide d'un agent de chloration choisi parmi un mélange chlore-eau, un mélange chlore-solution d'acide chlorhydrique, un mélange solution d'acide chlorhydrique-peroxyde d'hydrogène, dans un solvant miscible à l'eau ou à une solution d'acide chlorhydrique.

2. Procédé selon la revendication 1 caractérisé en ce que la quinone et ses dérivés éventuellement chlorés répondent à la formule (I)

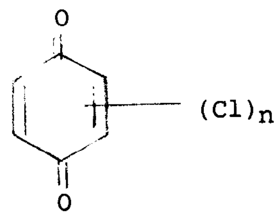

(I)

dans laquelle n est égal ou supérieur à 0 et inférieur ou égal à 3.

3. Procédé selon la revendication 1 caractérisé en ce que l'hydroquinone et ses dérivés éventuellement chlorés répondent à la formule (II)

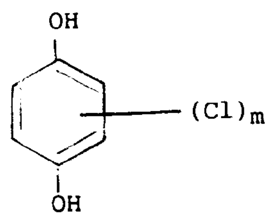

(II)

dans laquelle m est égal ou supérieur à 0 et inférieur ou égal à 4.

4. Procédé selon la revendication 1 caractérisé en ce que les solvants sont choisis parmi les alcools aliphatiques contenant 1 à 4 atomes de carbone, les acides carboxyliques contenant 1 à 4 atomes de carbone ainsi que leurs anhydrides, les amides les sulfoxides, l'éther isopropylique, le diméthyl sulfoxide, les nitriles.

5. Procédé selon la revendication 4 caractérisé en ce que les solvants sont choisis parmi l'éthanol et l'acide acétique.

## Patentansprüche

1. Verfahren zur Herstellung von Chloranil durch Chlorierung oder oxidierende Chlorierung von Chinonen, Hydrochinonen oder ihren Chlorderivaten in Abwesenheit eines Chlorierungskatalysators und unter Atmosphärendruck, dadurch gekennzeichnet, daß die Chlorierung mit Hilfe eines Chlorierungsmittels durchgeführt wird, das aus einem Chlor-Wasser-Gemisch, einem Gemisch von Chlor und einer Salzsäurelösung, einem Gemisch einer Salzsäurelösung mit einer Lösung von Wasserstoffperoxid, in einem mit Wasser oder einer Salzsäurelösung mischbaren Lösungsmittel ausgewählt ist.

2. Verfahren nach Anspruch 1, dadurch gekennzeichnet, daß das Chinon und dessen gegebenenfalls chlorierte Derivate der Formel (I)

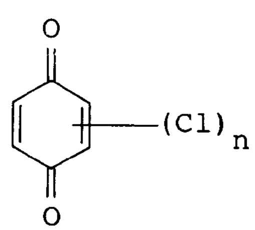

(I)

entsprechen, in der n gleich oder größer 0 und kleiner oder gleich 3 ist.

3. Verfahren nach Anspruch 1, dadurch gekennzeichnet, daß das Hydrochinon und seine gegebenenfalls chlorierten Derivate der Formel (II)

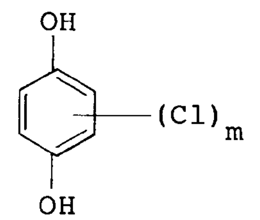

(II)

entsprechen, in der m gleich oder größer 0 und kleiner oder gleich 4 ist.

4. Verfahren nach Anspruch 1, dadurch gekennzeichnet, daß die Lösungsmittel aus aliphatischen Alkoholen mit 1 bis 4 Kohlenstoffatomen, Carbonsäuren mit 1 bis 4 Kohlenstoffatomen sowie ihren Anhydriden, Amiden, Sulfoxiden, Isopropylether, Dimethylsulfoxid und Nitrilen ausgewählt sind.

5. Verfahren nach Anspruch 4, dadurch gekennzeichnet, daß die Lösungsmittel aus Ethanol und Essigsäure ausgewählt sind.

**Claims**

1. Process for the preparation of chloranil by chlorination or oxychlorination of quinones, of hydroquinones or of their chlorinated derivatives in the absence of any chlorination catalyst and at atmospheric pressure, characterised in that the chlorination is carried out with the aid of a chlorinating agent chosen from a chlorine-water mixture, a chlorine-hydrochloric acid solution mixture or a hydrochloric acid solution-hydrogen peroxide mixture, in a solvent miscible with water or with a hydrochloric acid solution.

2. Process according to Claim 1, characterised in that the quinone and its optionally chlorinated derivatives correspond to the formula (I)

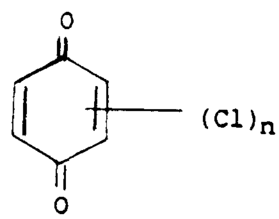

(I)

in which n is equal to or greater than 0 and smaller than or equal to 3.

3. Process according to Claim 1, characterised in that the hydroquinone and its optionally chlorinated derivatives correspond to the formula (II)

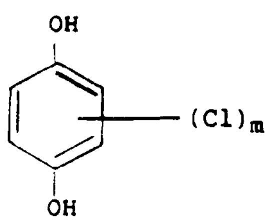

(II)

in which m is equal to or greater than 0 and smaller than or equal to 4.

4. Process according to Claim 1, characterised in that the solvents are chosen from aliphatic alcohols containing 1 to 4 carbon atoms, carboxylic acids containing 1 to 4 carbon atoms and their anhydrides, amides, sulphoxides, isopropyl ether, dimethyl sulphoxide and nitriles.

5. Process according to Claim 4, characterised in that the solvents are chosen from ethanol and acetic acid.